# EUROPEAN PATENT APPLICATION

(11) **EP 3 208 927 A1**
(43) Date of publication of application: **23.08.2017**
(21) Application number: 15849932.7
(22) Date of filing: 18.09.2015
(51) Int. Cl.: H02M 7/12, C07C 251/44, H01L 33/00, C07B 61/00, C07D 201/04, C07D 223/10, C07D 225/02

(54) **THREE-PHASE AC/DC CONVERSION DEVICE, PHOTOCHEMICAL REACTION DEVICE AND METHOD USING SAME, AND METHOD FOR PRODUCING LACTAM**

(30) Priority: 15.10.2014 JP 2014210567
(71) Applicant: Toray Industries, Inc., Tokyo 103-8666 (JP)
(72) Inventor: OHNO, Fumikatsu, Otsu-shi Shiga 5208558 (JP); TAKAHASHI, Toru, Otsu-shi Shiga 520-8558 (JP); ITO, Hiroyasu, Nagoya-shi Aichi 4558502 (JP); SUGAWARA, Kazuki, Tokai-shi Aichi 476-8567 (JP)
(74) Representative: Hoefer & Partner Patentanwälte mbB
(86) International application number: PCT/JP2015/076613
(87) International publication number: WO 2016/059942

(57) **Abstract**

Provided are a three-phase AC/DC converter disposed between a three-phase AC power supply and a light emitting diode group, the converter comprising: a three-phase full bridge circuit in which pairs of switching elements are connected in parallel between DC buses for the three phases of the three-phase AC power supply; reactors connecting connection portions between the switching elements and corresponding phases of the three-phase AC power supply; a smoothing capacitor on the output side of the three-phase full bridge circuit; a DC voltage detection means; a power supply voltage phase detection means; and a pulse width modulation means for outputting pulse width modulation signals of the switching elements, wherein the pulse width modulation means outputs the pulse width modulation signals based on a power supply voltage phase and an output voltage between the DC buses, a photochemical reaction device and a photochemical reaction method using the device, and a method for producing lactam using the photochemical reaction method. The present invention makes it possible to cause the light emitting diode group having a large capacity to stably and continuously illuminate while suppressing an occurrence of a voltage drop phenomenon and suppressing an influence on the AC power supply side, thereby contributing to the stabilization of the photochemical reaction.

## Description

### Technical Field of the Invention

The present invention relates to a three-phase AC/DC converter for driving a group of light emitting diodes (hereinafter, also abbreviated as LEDs), photochemical reaction device and method using the three-phase AC/DC converter, and a method for producing a lactam.

### Background Art of the Invention

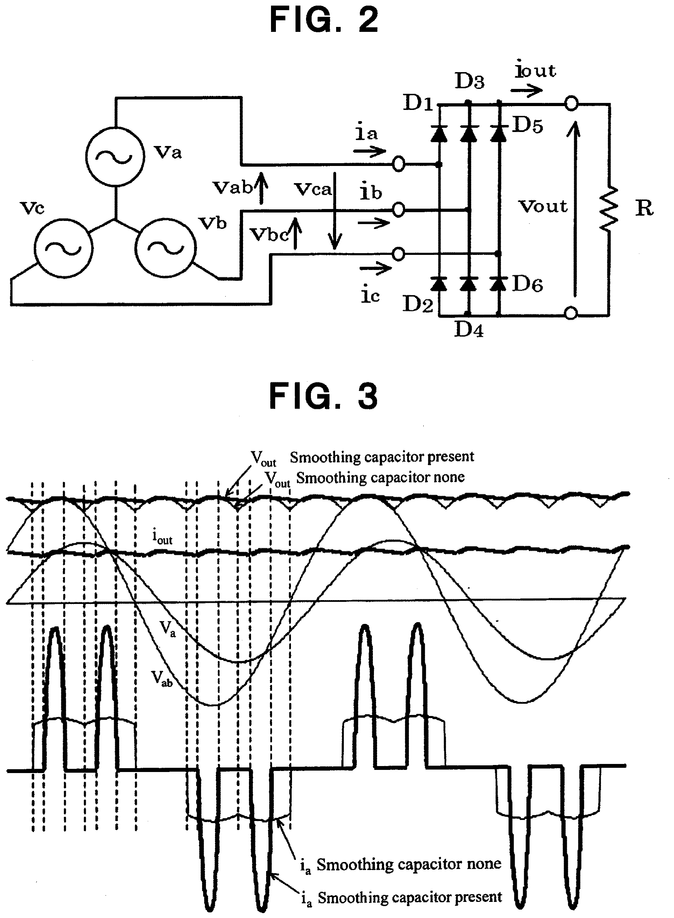

Conventionally, a three-phase full-wave rectification circuit is being used for rectification of three-phase alternating current, and this has been carried out, for example, as shown in Fig. 2, by a circuit incorporated so that it continues to always output a highest voltage as the output voltage Vₒᵤₜ by using a three-phase bridge circuit incorporating switching elements (D₁ to D₆) and flowing a current (any one of iₐ, i_{b}, i_{c}), from a circuit with the highest voltage among the voltages (V_{ab}, V_{bc}, V_{ca}) of the three phases (Va to Vc), as the output current iₒᵤₜ.

As a result, as shown in Fig. 3, although relatively constant current and voltage can be obtained as the output current iₒᵤₜ and the output voltage Vₒᵤₜ, the waveform of currens iₐ, i_{b} or i_{c} (only iₐ is shown for easy understanding) is far away from a sine wave that should be originally formed, and there is a problem that the power factor (electricity that can be used effectively) is reduced.

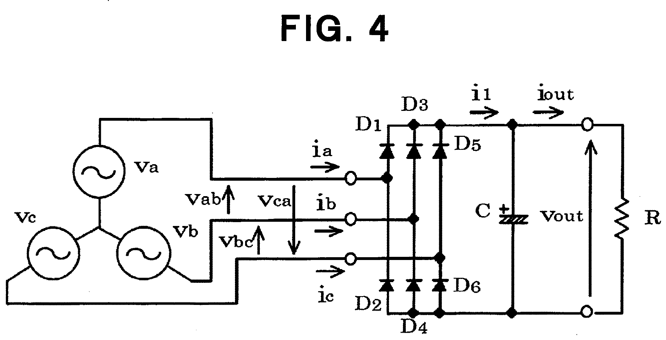

Further, as shown in Fig. 4, in order to smooth the current and voltage on the output side, a smoothing capacitor C is added to the output side to form as a three-phase full-wave rectification circuit with a smoothing capacitor as shown in the figure. According to this, although the output waveform is made more constant as shown in Fig. 3, because the electricity stored in the smoothing capacitor C returns to the AC side through the circuit which is most likely to flow at that time, this waveform becomes a rabbit's ear type, it may cause troubles to alternating current due to higher harmonics.

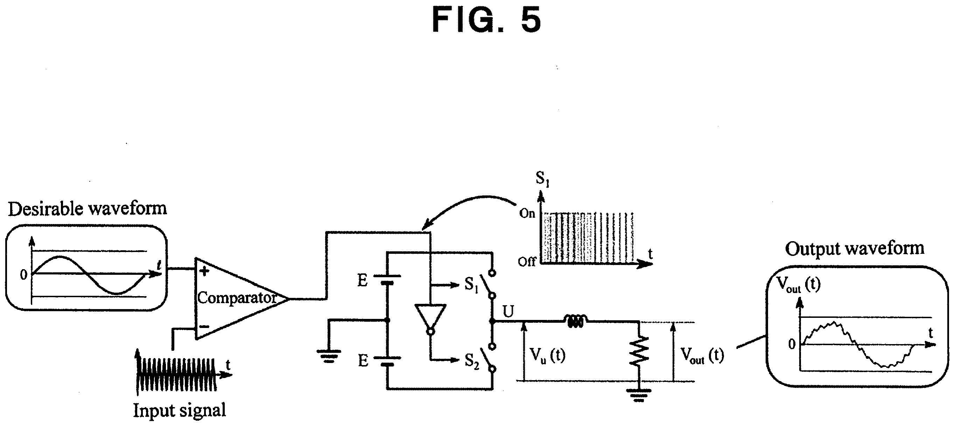

Therefore, in order to cope with the above-described problem, it is known to use a pulse width modulation (hereinafter, also abbreviated as PWM) converter technology (for example, Patent documents 1 and 2). This is, for example, a technology as described below. Fig. 5 exemplifies an inverter driving circuit due to PWM control. PWM signal is a technology for outputting a desired waveform by comparing a waveform which is an input signal and a preset desired waveform and determining a difference therebetween, and by deciding the ON/OFF timing of switches S1 and S2 and the width thereof. As shown in the figure, an output waveform close to the desired waveform set on the input side can be obtained. By applying such a PWM technology to a converter and applying it to a three-phase full-wave rectification circuit as shown in Fig. 2 or Fig. 4, each of waveforms of iₐ, i_{b} and i_{c} is set as a sine wave, and it is controlled by a computer so that a phase difference with a voltage of the primary AC side is minimized. Namely, usually rectification is performed by a bridge circuit and a capacitor, but in this control, by rectifying by using a switching element due to PWM control therein, while rectification on the secondary side is performed, switching for constant voltage control is performed so that the current waveform on the primary side is synchronized with the primary voltage, and this control makes it possible to eliminate waveform distortion such as higher harmonics, noise, high frequency and the like on the primary side. By this, it becomes possible to remove higher harmonics and noise and the like superimposed on the transformer on the primary side. Further, since it is possible to control the output voltage on the secondary side constant, the accuracy of the constant voltage on the secondary side can be improved. As a result, because it becomes unnecessary to take countermeasures against higher harmonics and high-frequency correspondence on the primary side, it becomes possible to make the transformer on the primary side a sine wave, and it becomes possible to suppress the voltage drop.

### Prior art documents

### Patent documents

Patent document 1: WO2012/049706
Patent document 2: WO 2008/108147

### Summary of the Invention

### Problems to be solved by the Invention

Although a DC power supply is required to drive the LED and it is indispensable to maintain the DC voltage fluctuation range within the LED illuminating voltage range from the characteristics of the LED, because the range is narrow, an accuracy necessary for maintaining the voltage is required. In order to drive a large amount of LEDs, for example, in order to drive a large amount of LED groups, a large number of constant current devices are required, but in such a condition, waveform distortion due to noise, high frequency or the like increases. If a general diode converter is used as a three-phase AC/DC converter for driving a group of LEDs, higher harmonics, high-frequency waves and noise are generated in the transformer on the primary side, thereby causing a drop in power supply voltage. As a result, the output voltage on the secondary side for driving the LED group also decreases, consequently there is a possibility causing a situation that the voltage drops below the minimum voltage required for the LED group, and therefore, there is a possibility that a turning off or flashing phenomenon of the LED occurs, which makes it difficult to maintain continuous illuminating.

Accordingly, an object of the present invention is to provide a three-phase AC/DC converter which can be used for driving a large-capacity, particularly 3 kw or more, light emitting diode group with a single unit, and which can eliminate the possibility of turning off or flashing phenomenon of the LED.

It is another object of the present invention to provide photochemical reaction device and method having a photoirradiation device provided with the above-described three-phase AC/DC converter, and a method for producing a lactam using the photochemical reaction method.

### Means for solving the Problems

To achieve the above-described objects, a three-phase AC/DC converter according to the present invention is a device incorporated into a power supply circuit disposed between a three-phase AC power supply and a light emitting diode group, in order to drive the light emitting diode group of 3 kw or more with a single unit, and is characterized in that the converter comprises:
DC buses connected to the light emitting diode group;
a three-phase full bridge circuit in which pairs of switching elements, in each of which a pair of switching elements are connected in series, are connected in parallel between the DC buses by pairs for three phases of the three-phase AC power supply, and each switching element has a reverse-blocking diode connected thereto in parallel;
a reactor provided between the three-phase full bridge circuit and the three-phase AC power supply for connecting a connection portion between switching elements in the each pair of switching elements and a corresponding phase of the three-phase AC power supply;
a smoothing capacitor connected between the DC buses on an output side of the three-phase full bridge circuit;
a DC voltage detection means for detecting an output voltage between the DC buses;
a power supply voltage phase detection means for detecting a power supply voltage phase of the three-phase AC power supply; and
a pulse width modulation means for outputting a pulse width modulation signal for controlling each of the switching elements,
wherein the pulse width modulation means outputs the pulse width modulation signal based on the power supply voltage phase and the output voltage between the DC buses.

In such a three-phase AC/DC converter according to the present invention, by using a converter comprising a three-phase full bridge circuit, in which switching elements capable of performing PWM control are combined, to a converting part into a direct current, it becomes possible to correct the high frequency and noise generated on the secondary side and the high frequency generated on the primary side to obtain a power supply waveform with no distortion, and the voltage drop at the transformer (reactor) on the primary side is suppressed. Further, by performing the constant voltage control that can control the DC voltage on the secondary side to be constant, and besides, by making it possible to supply a more stable voltage to the secondary side by applying PWM control to the three-phase full bridge circuit in which switching elements are combined and adding the smoothing capacitor to the output side, it is also possible to solve the problem of a slight drop of the secondary voltage, and by eliminating the possibility of occurrence of turning off or flashing phenomenon of the LED accompanying with the voltage drop, it also becomes possible to continuously illuminate a large-sized LED module of, for example, 10 kw or more.

In the above-described three-phase AC/DC converter according to the present invention, an embodiment can be employed wherein a plurality of constant current circuits each for controlling a current to the light emitting diode group constant are provided in parallel on an output side of the three-phase full bridge circuit. The plurality of constant current circuits may be disposed in accordance with the disposition formation of light emitting diode groups, and may be disposed at a portion immediately before each light emitting diode group of the power supply circuit to each light emitting diode group. As the constant current circuit, any of generally used ones can be used, and a commercially available constant current circuit can be used.

Further, in the three-phase AC/DC converter according to the present invention, in order to ensure a more stable illuminating operation of the light emitting diode group, it is preferred that the output voltage between the DC buses is 100V or more, and a voltage drop thereof is controlled to 10% or less. In particular, by suppressing the voltage drop to 10% or less, it becomes possible to stably and continuously illuminate even in a large-sized LED module.

Similarly, in order to ensure a more stable illuminating operation of the large-capacity light emitting diode group, with respect to the above-described plurality of constant current circuits provided in parallel, it is preferred that one constant current circuit can control a constant current of 1 ampere or more.

A photochemical reaction device according to the present invention is characterized by having a photoirradiation device comprising a light emitting diode group connected to the above-described three-phase AC/DC converter. By applying the above-described three-phase AC/DC converter, it becomes possible to stably and continuously illuminate the light emitting diode group, and it becomes possible to perform a desired photochemical reaction due to the photoirradiation device.

Further, a photochemical reaction method according to the present invention comprises a method characterized by using such a photochemical reaction device.

This photochemical reaction method according to the present invention can be applied to any of photochemical reactions required to stably and continuously illuminate a large-capacity light emitting diode group, for example, to a photochemical reaction in which the destination of photoirradiation is a liquid, and the composition of thee liquid contains at least a carbon atom. As the liquid as the destination of photoirradiation, a cycloalkane can be exemplified. As the cycloalkane, cyclohexane or cyclododecane can be exemplified. The photochemical reaction method according to the present invention is suitable to, in particular, a photochemical reaction method wherein a cycloalkanone oxime is produced by performing photoirradiation to such a cycloalkane and a photo nitrosating agent. As the photo nitrosating agent, nitrosyl chloride or trichloronitrosomethane can be exemplified.

A method for producing a lactam according to the present invention is characterized by using a cycloalkanone oxime produced by the photochemical reaction method as described above.

### Effect according to the Invention

In the three-phase AC/DC converter according to the present invention, when light-emitting body using a large-capacity light emitting diode group is served to light emitting, it becomes possible to stably and continuously illuminate the light emitting diode group while suppressing occurrence of the voltage drop phenomenon and also suppressing an influence to the AC power source side. Therefore, this three-phase AC/DC converter is particularly effective for photochemical reaction device and method performing photoirradiation by using a large-capacity light emitting diode group, and further, can contribute to stabilize the method for producing a lactam using a cycloalkanone oxime produced by the photochemical reaction method.

### Brief explanation of the drawings

[Fig. 1] Fig. 1 is a circuit diagram of a three-phase AC/DC converter according to an embodiment of the present invention.
[Fig. 2] Fig. 2 is a circuit diagram exemplifying a conventional three-phase full-wave rectification circuit.
[Fig. 3] Fig. 3 is a waveform diagram exemplifying an output waveform due to the rectification circuit shown in Figs. 2 and 4.
[Fig. 4] Fig. 4 is a circuit diagram exemplifying a case where a smoothing capacitor is added to the three-phase full-wave rectification circuit shown in Fig. 2.
[Fig. 5] Fig. 5 is a circuit explanatory diagram exemplifying and explaining an inverter driving circuit according to a conventional PWM control.

### Embodiments for carrying out the Invention

Hereinafter, embodiments of the present invention will be explained referring to figures.

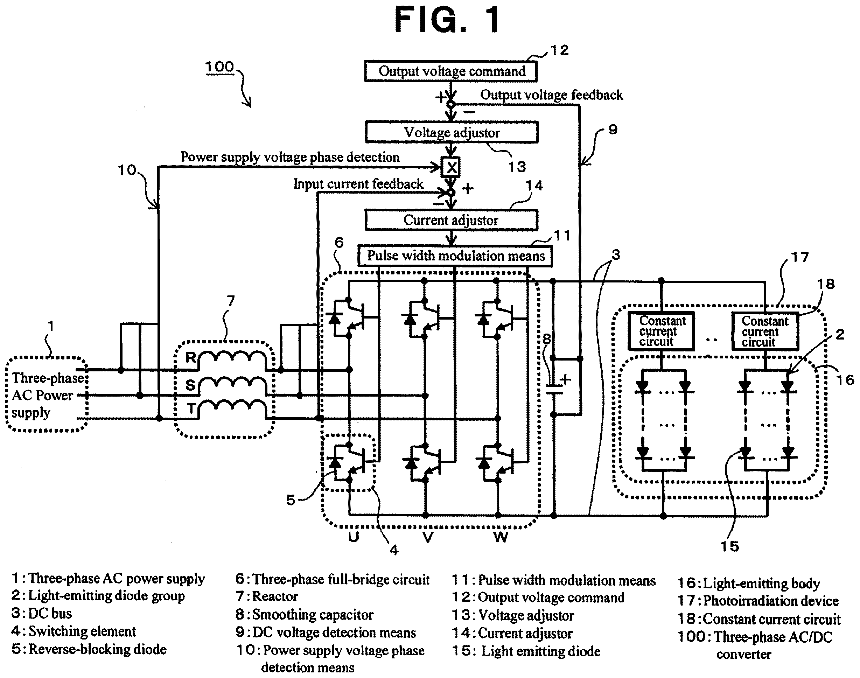

Fig. 1 shows a circuit of a three-phase AC/DC converter according to an embodiment of the present invention. The three-phase AC/DC converter 100 shown in Fig. 1 is a three-phase AC/DC converter incorporated into a power supply circuit disposed between a three-phase AC power supply 1 and light emitting diode groups 2, in order to drive the light emitting diode groups of 3 kw or more with a single unit. The three-phase AC/DC converter 100 has DC buses 3 connected to the light emitting diode groups 2; a three-phase full bridge circuit 6 (bridge circuit of three phases U, V and W) in which pairs of switching elements 4, in each of which a pair of switching elements 4 are connected in series, are connected in parallel between the DC buses 3 by pairs for three phases of the three-phase AC power supply 1, and each switching element 4 has a reverse-blocking diode 5 connected thereto in parallel; a reactor 7 provided between the three-phase full bridge circuit 6 and the three-phase AC power supply 1 for connecting a connection portion between switching elements 4 in each pair of switching elements 4 and a corresponding phase (phase R, S or T) of the three-phase AC power supply 1; a smoothing capacitor 8 connected between the DC buses 3 on an output side of the three-phase full bridge circuit 6; a DC voltage detection means 9 for detecting an output voltage between the DC buses 3; a power supply voltage phase detection means 10 for detecting a power supply voltage phase of the three-phase AC power supply 1; and a pulse width modulation means (PWM means) 11 connected to each of the switching elements 4 for outputting a pulse width modulation signal for controlling each of the switching elements 4. The pulse width modulation means 11 outputs the pulse width modulation signal to each of the switching elements 4 based on the power supply voltage phase detected by the power supply voltage phase detection means 10 and the output voltage between the DC buses 3 detected by the DC voltage detection means 9.

In this embodiment, the output voltage between the DC buses 3 detected by the DC voltage detection means 9 and fed back and a preset output voltage command 12 are compared, and adjusted by a voltage adjuster 13. The current based on the phase of the adjusted voltage and the power supply voltage phase detected by the power supply voltage phase detection means 10 is compared with the input current fed back from the input side of the three-phase full bridge circuit 6, and after the current is adjusted by a current adjustor 14, it is subjected to the pulse width modulation control due to the pulse width modulation means 11.

Further, in this embodiment, a plurality of light emitting diodes 15 are combined and connected to form one light emitting diode group 2, a plurality of light emitting diode groups 2 are provided in parallel, and a large-scale light-emitting body 16 is constituted. A device having this light-emitting body 16 is configured as a photoirradiation device 17 used in, for example, a photochemical reaction device. In this photoirradiation device 17, a plurality of constant current circuits 18 for controlling the currents to the respective light emitting diode groups 2 to be constant are provided in parallel relatively to the output side of the three-phase full bridge circuits 6.

In the three-phase AC/DC converter 100 thus constructed, since a converter comprising the three-phase full bridge circuit 6 combined with switching elements 4 capable of being performed with PWM control is formed on the converting section from three-phase AC to DC, it becomes possible to correct the high frequency and noise on the secondary side, that is, the output side (DC buses 3 side) of the three-phase full bridge circuit 6, and the high frequency generated on the primary side, and make it a power supply waveform having no distortion, and the voltage drop on the primary side, that is, on the input side (reactor 7 side) of the 3-phase full bridge circuit 6 is suppressed. Further, since the smoothing capacitor 8 is also added, the DC voltage on the side of the DC buses 3 is controlled at a constant voltage with a smooth waveform, and by applying the PWM control to the three-phase full bridge circuit 6, a stable voltage supply with less fluctuation becomes possible. In case where the output voltage between the DC buses 3 is 100 V or more, it becomes possible to easily suppress the voltage drop at 10% or less, and it becomes possible to eliminate the possibility of turning off or flashing phenomenon of the LED accompanying the voltage drop, and to stably and continuously illuminate a large-sized LED module (light-emitting body 16) of, for example, 10 kw or more. Furthermore, since the constant current circuit 18 (for example, a constant current circuit capable of controlling a constant current of 1 ampere or more) is provided for each light emitting diode group 2, the current supplied to each light emitting diode group 2 is stabilized, ultimately, the power supply to the entire light-emitting body 16 is stabilized, and a stable and continuous illumination becomes possible even in a large-capacity light-emitting body 16.

The photoirradiation device 17 including the light emitting diode group 2 connected to the above-described three-phase AC/DC converter 100 can be applied to various kinds of photochemical reaction devices, and with a single three-phase AC/DC converter 100, even in case of large-capacity light emitting diode groups 2, it becomes possible to stably and continuously illuminate the whole of the light emitting diode groups 2, and it becomes possible to stably perform a desirable photochemical reaction by the photoirradiation device 17.

Such a photochemical reaction device can be used for various kinds of photochemical reaction methods, and in particular, can be applied to any photochemical reaction which is required to stably and continuously illuminate the large-capacity light emitting diode group 2. For example, in the photochemical reaction method, the destination of the photoirradiation can be set to be a liquid which contains carbon atoms. Namely, in the photochemical reaction method according to the present invention, at least one destination of the photoirradiation may be a raw material system composed of a liquid. The liquid served as a raw material is not particularly restricted as long as it is a liquid containing carbon atoms, and as a reaction liquid, a flammable liquid, for example, hydrocarbons such as alkane and cycloalkane can be exemplified.

In the present invention, although the cycloalkane is not particularly limited in the number of carbon atoms, for example, preferred are cyclopropane, cyclobutane, cyclopentane, cyclohexane, cycloheptane, cyclooctane, cyclononane, cyclodecane, cycloundecane, and cyclododecane. In particular, cyclohexane as a raw material of caprolactam and cyclododecane as a raw material of lauryllactam are preferred.

Using the above-described cycloalkane and a photo nitrosating agent, cycloalkanone oxime is obtained by photochemical reaction due to the photo irradiation from the photoirradiation device 17 as a light source. As the photo nitrosating agent, for example, nitrosyl chloride or a mixed gas of nitrosyl chloride and hydrogen chloride is preferable. Besides, since any of the mixed gas of nitric monoxide and chlorine, the mixed gas of nitric monoxide, chlorine and hydrogen chloride, the mixed gas of nitrose gas and chlorine, etc. acts as nitrosyl chloride in the photochemical reaction system, it is not limited to these supply forms of the nitrosating agent. Further, trichloronitrosomethane obtained by photochemical reaction of nitrosyl chloride and chloroform may be used as a nitrosating agent. When the photochemical reaction is carried out in the presence of hydrogen chloride, the cycloalkanone oxime becomes its hydrochloride, but it may be in the form of hydrochloride as it is.

By the above-described photochemical reaction, it is possible to obtain cycloalkanone oxime which depends upon the carbon number of the cycloalkane. For example, cyclohexanone oxime is obtained by photo nitrosating reaction with nitrosyl chloride using cyclohexane. Further, cyclododecanone oxime is obtained by photo nitrosating reaction with nitrosyl chloride using cyclododecane.

A lactam can be obtained by Beckmann rearrangement of the cycloalkanone oxime obtained by the photochemical reaction. For example, in the reaction of Beckmann rearrangement of cyclohexanone oxime, ε-caprolactam is obtained as shown by the following reaction formula [Chemical formula 1]. Further, co-laurolactam is obtained in the reaction of Beckmann rearrangement of cyclododecanone oxime.

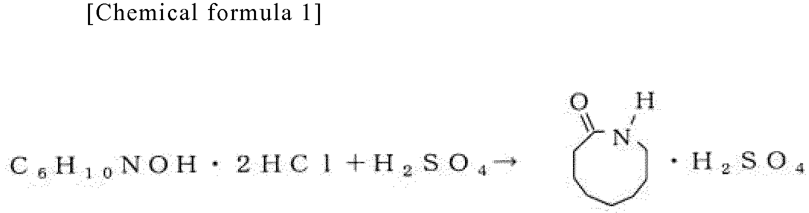

In the above description, although the embodiment of the present invention has been explained with reference to Fig. 1, this embodiment is shown as an example, and it is not intended to limit the scope of the present invention. It can be carried out in various forms, and can be simplified or changed without departing from the gist of the present invention. These embodiments and modifications thereof are also included in the scope of the present invention.

### Industrial Applicability

The present invention can be applied to a three-phase AC/DC converter in any field where stable power supply to a large-capacity light emitting diode group is required, and in particular, can be applied to any photochemical reaction using a large-scale photoirradiation device, and particularly, it is useful to be applied for production of cycloalkanone oxime and production of lactam.

### Explanation of symbols

- 1:: three-phase AC power supply
- 2:: light emitting diode group
- 3:: DC bus
- 4:: switching element
- 5:: reverse blocking diode
- 6:: three-phase full bridge circuit
- 7:: reactor
- 8:: smoothing capacitor
- 9:: DC voltage detection means
- 10:: power supply voltage phase detection means
- 11:: pulse width modulation means
- 12:: output voltage command
- 13:: voltage adjustor
- 14:: current adjustor
- 15:: light emitting diode
- 16:: light-emitting body
- 17:: photoirradiation device
- 18:: constant current circuit
- 100:: three-phase AC/ DC converter

## Claims

1. A three-phase AC/DC converter incorporated into a power supply circuit disposed between a three-phase AC power supply and a light emitting diode group, in order to drive said light emitting diode group of 3 kw or more with a single unit, **characterized in that** said converter comprises:
DC buses connected to said light emitting diode group;
a three-phase full bridge circuit in which pairs of switching elements, in each of which a pair of switching elements are connected in series, are connected in parallel between said DC buses by pairs for three phases of said three-phase AC power supply, and each switching element has a reverse-blocking diode connected thereto in parallel;
a reactor provided between said three-phase full bridge circuit and said three-phase AC power supply for connecting a connection portion between switching elements in said each pair of switching elements and a corresponding phase of said three-phase AC power supply;
a smoothing capacitor connected between said DC buses on an output side of said three-phase full bridge circuit;
a DC voltage detection means for detecting an output voltage between said DC buses;
a power supply voltage phase detection means for detecting a power supply voltage phase of said three-phase AC power supply; and
a pulse width modulation means for outputting a pulse width modulation signal for controlling each of said switching elements,
wherein said pulse width modulation means outputs said pulse width modulation signal based on said power supply voltage phase and said output voltage between said DC buses.

2. The three-phase AC/DC converter according to claim 1, wherein a plurality of constant current circuits each for controlling a current to said light emitting diode group constant are provided in parallel on an output side of said three-phase full bridge circuit.

3. The three-phase AC/DC converter according to claim 1 or 2, wherein said output voltage between said DC buses is 100V or more, and a voltage drop thereof is controlled to 10% or less.

4. The three-phase AC/DC converter according to claim 2 or 3, wherein one constant current circuit controls a constant current of 1 ampere or more.

5. A photochemical reaction device **characterized by** having a photoirradiation device comprising a light emitting diode group connected to the three-phase AC/DC converter according to any one of claims 1 to 4.

6. A photochemical reaction method **characterized by** using the photochemical reaction device according to claim 5.

7. The photochemical reaction method according to claim 6, wherein the destination of photoirradiation is a liquid, and the composition of said liquid contains at least a carbon atom.

8. The photochemical reaction method according to claim 7, wherein said liquid as the destination of photoirradiation is a cycloalkane.

9. The photochemical reaction method according to claim 8, wherein said cycloalkane is cyclohexane or cyclododecane.

10. The photochemical reaction method according to claim 8 or 9, wherein a cycloalkanone oxime is produced by performing photoirradiation to said cycloalkane and a photo nitrosating agent.

11. The photochemical reaction method according to claim 10, wherein said photo nitrosating agent is nitrosyl chloride or trichloronitrosomethane.

12. A method for producing a lactam **characterized by** using a cycloalkanone oxime produced by the photochemical reaction method according to claim 10 or 11.
